(19) Europäisches Patentamt
European Patent Office
Office européen des brevets

(11) Publication number: **0 367 944 A2**

(12) **EUROPEAN PATENT APPLICATION**

(21) Application number: **89116865.0**

(22) Date of filing: **12.09.89**

(51) Int. Cl.[5]: **C07D 239/90, C07C 43/225, C07C 39/21, A61K 31/505**

(30) Priority: **01.11.88 CS 7195/88**

(43) Date of publication of application: **16.05.90 Bulletin 90/20**

(84) Designated Contracting States: **AT BE CH DE FR GB IT LI LU NL SE**

(71) Applicant: **SPOFA spojené podniky pro zdravotnickou vyrobu**
**No 11 Husinecká**
**Praha 3(CS)**

(72) Inventor: **Fisnerová, Ludmila, Dipl. Ing.**
**Zvonková 2640**
**Praha 10(CS)**
Inventor: **Brunová, Bohumila, Dipl. Ing.**
**Ostrovského 33**
**Praha 5(CS)**
Inventor: **Maturová, Eva, Dr.**
**Zvonková 2**
**Praha 10(CS)**
Inventor: **Grimová, Jaroslava, Dr.**
**Na Doubkové 2**
**Praha 5(CS)**

(74) Representative: **Patentanwälte Beetz sen. - Beetz jun. Timpe - Siegfried - Schmitt-Fumian**
**Steinsdorfstrasse 10**
**D-8000 München 22(DE)**

(54) **Ether derivative of 4-[3H] -quinazolinone, a process for the preparation thereof, and pharmaceutical compositions.**

(57) The invention relates to 3-[2-(2′,4′-difluoro-4-biphenylyloxy)-ethyl]-4-[3H]-quinazolinone and its acid addition salts, particularly the hydrochloride, to a process for preparing these compounds and intermediates thereof, and to pharmaceutical compositions comprising these compounds as active ingredients. The ether derivative of 4-[3H]-quinazolinone according to the invention possesses considerably higher analgesic activity and lower acute toxicity than aminophenazone, ibuprofen, acetylosalicylic acid and paracetamol.

EP 0 367 944 A2

## Ether derivative of 4-[3H]-quinazolinone, a process for the preparation thereof, and pharmaceutical compositions

The invention relates to 3-[2-(2′,4′-difluoro-4-biphenylyloxy)-ethyl]-4-[3H]-quinazolinone of formula I

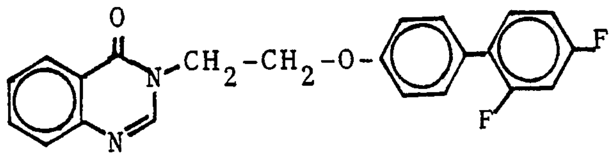

(I) ,

which is an ether derivative of 4-[3H]-quinazolinone, and its addition salts, particularly with pharmaceutically acceptable organic and inorganic acids, preferably its hydrochloride, a method for the preparation of these compounds and their intermediates, and to pharmaceutical compositions comprising these compounds of formula I as active ingredients.

The compound of formula I and its salts show a remarkable analgesic activity and a substantially lower toxicity than aminophenazone (1-phenyl-2,3-dimethyl-4-dimethylamino-5-pyrazolone), ibuprofen, acetylosalicylic acid and paracetamol.

The process for the preparation of compound I and its acid addition salts principally consists in the reaction of 4-[3H]-quinazolinone with the respective halogenated ether, i.e. 2′,4′-difluoro-4-(2-chloroethoxy)-biphenyl of formula II

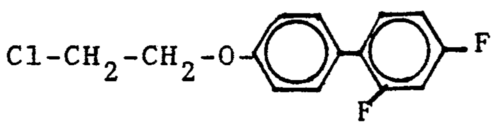

(II).

The reaction is conducted in the presence of sodium hydride in an inert organic solvent, such as e.g. dimethylformamide, advantageously at a temperature of from 90 to 110 °C. If required, the resulting base of formula I is converted by neutralization with an appropriate organic or inorganic acid, e.g. hydrochloric acid, into the respective addition salts which are suitable for the formulation of pharmaceutical dosage forms and compositions and for conducting biological assays.

The starting compound 4-[3H]-quianzolinone is a known substance which may be prepared by methods described in the pertinent literature. The second starting material, the compound of formula II, is a novel compound which can be prepared by reacting the respective substituted 4-hydroxybiphenyl derivative, i.e. 2′,4′-difluoro-4-hydroxybiphenyl (compound of formula III),

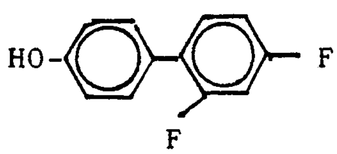

(III).

with 2-chloroethyl p-toluenesulfonate (cf. G.R. Clemo, W.H. Perkin, J. Chem. Soc. 1922, 642).

The subject compound of formula I which has been selected from a larger number of related structures under preliminary pharmacological evaluation, proved, especially in the form of its hydrochloride, to possess remarkable properties of medicinal importance, particularly analgesic activity, which allow a useful application of this compound in pharmacology, particularly as a potent, safe analgesic agent.

Known analgesic drugs heretofore in use, such as e.g. aminophenazone, phenacetin, acetylosalicylic acid and the like, exhibit certain undesired adverse side effects. Thus, extensive investigations of recent years have shown inter alia that aminophenazone, which is currently employed as a standard reference compound in pharmacological tests for analgesic activity, has potential cancerogenic properties, as a result of which the use of this compound has been prohibited or substantially restricted in many countries.

The analgesic activity and acute (short-time) toxicity data ($ED_{50}$ and $LD_{50}$, respectively) of compound I

(hydrochloride), and of aminophenazone, ibuprofen, acetylosalicylic acid and paracetamol taken as reference compounds for comparison are summarized in the following table.

| Compound | $ED_{50}$ (mg/kg, p.o.) | $LD_{50}$ (mg/kg, p.o.) |
|---|---|---|
| Compound of formula I (base) | 71 | > 1000 |
| Compound of formula I (hydrochloride) | 26 | > 2000 |
| Aminophenazone | 104 | 800 |
| Ibuprofen [a] | 179 | 1258 |
| Acetylosalicylic acid | 190 | 1068 |
| Paracetamol [b] | 285 | 1088 |

[a] 2-(4-isobutylphenyl)-propionic acid
[b] 4-hydroxyacetanilide.

The subject compound of formula I and its salts are proved to be free of any signs of gastrotoxicity and cancerogeneity.

The following example relates to the preparation of the compound of formula I and its salts and to the preparation of intermediates.

## Example

1. Preparation of 2′,4′-difluoro-4-(2-chloroethoxy)-biphenyl

### Procedure A

A mixture of 58,5 g of 2′,4′-difluoro-4-hydroxybiphenyl (4-(2′,4′-difluorophenyl)-phenol), 13 g of sodium hydroxide, 26 ml of water and 66 g of 2-chloroethyl p-toluenesulfonate is stirred for 2 hours at a temperature of 95 to 100 °C. After cooling to 20 to 25 °C the reaction mixture is diluted with water, and the product is collected on a filter and crystallized from aqueous ethanol (2:1) to give 35 g (yield 45 % of theory) of the title compound, m.p. 72 to 74 °C.

### Procedure B

To a solution of 15 g of sodium hydroxide in 30 ml of water are added 68,2 g of 4-(2′,4′-difluorophenyl)-phenol and 106 g of 2-chloroethyl p-toluenesulfonate, and the mixture is heated with stirring to a temperature of 95 to 100 °C (at 96 to 98 °C the exothermic reaction occurs) and maintained at this temperature for 5 hours. The reaction mixture is then diluted with dichloromethane, and the solution is washed with water and subsequently evaporated to dryness. The crude product is crystallized from isopropanol to yield 62 g (69 % of theory) of 2′,4′-difluoro-4-(2-chloroethoxy)-biphenyl, m.p. 72 to 73 °C. A further fraction (9 g) of pure product can be obtained from the mother liquor, the total yield of the product being 80 % of theory.

2. Preparation of 3-[2-(2′,4′-difluoro-4-biphenylyloxy)-ethyl]-4-[3H]-quinazolinone

To a solution of 8,76 g of 4-[3H]-quinazolinone in 120 ml of dimethylformamide 2 g of 80 % sodium hydride are gradually and portionwise added at an initial temperature of 25 °C. The mixture is then heated with stirring to 100 °C, maintained at this temperature for 15 minutes and cooled to 50 °C, whereupon 16,1 g of 2′,4′-difluoro-4-(2-chloroethoxy)-biphenyl are added in a single portion. The mixture is heated up again and maintained for 2,5 hours at a temperature of 98 to 105 °C. After cooling to 20 to 25 °C the mixture is

diluted with water, and the crude product is separated, dried and crystallized from ethyl acetate to yield 16,7 g of the base, m.p. 183 to 184 °C.

The afore mentioned base is converted into the hydrochloride by the following procedure: A solution of 16,7 g of the base prepared as above in 185 ml of chloroform is saturated with dry hydrogen chloride gas, and the mixture is allowed for 12 hours at a temperature of 20 to 22 °C to crystallize to give 18 g of the desired hydrochloride.

## Claims

1. 3-[2-(2′,4′-Difluoro-4-biphenylyloxy)-ethyl]-4-[3H]-quinazolinone of formula I

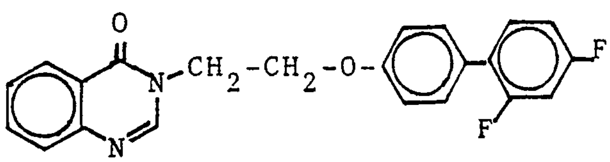

(I) ,

and its acid addition salts.

2. 3-[2-(2′,4′-Difluoro-4-biphenylyloxy)-ethyl]-4-[3H]-quinazolinone hydrochloride.

3. A process for the preparation of 3-[2-(2′,4′-difluoro-4-biphenylyloxy)-ethyl]-4-[3H]-quinazolinone of formula I and its acid addition salts according to claim 1 or 2,
characterized by
reaction of 4-[3H]-quinazolinone with 2′,4′-difluoro-4-(2-chloroethoxy)-biphenyl of formula II

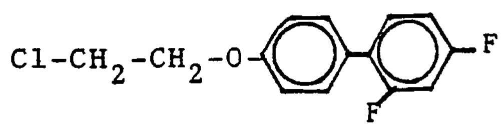

(II)

and optionally subsequent conversion of the resulting base into an acid addition salt thereof.

4. The process according to claim 3, characterized in that the reaction is conducted in the presence of sodium hydride in an inert organic solvent at a temperature of from about 90 to about 110 °C.

5. Pharmaceutical compositions, characterized in that they comprise 3-[2-(2′,4′-difluoro-4-biphenylyloxy)-ethyl]-4-[3H]-quinazolinone of formula I and/or a pharmaceutically acceptable acid addition salt thereof according to claim 1 or 2.

6. 2′,4′-Difluoro-4-(2-chloroethoxy)-biphenyl of formula II

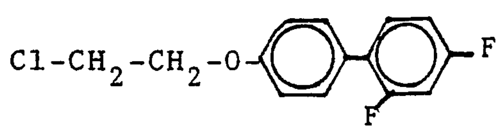

(II).

7. 2′,4′-Difluoro-4-hydroxybiphenyl of formula III

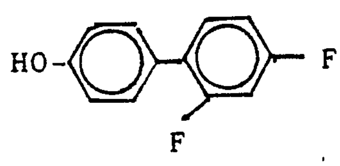

(III).

8. A process for the preparation of the compound of formula II according to claim 6, characterized by reaction of the compound of formula III according to claim 7 with 2-chloroethyl p-toluene sulfonate.

9. The process according to claim 8, characterized in that the reaction of the compound of formula II with 2-chloroethyl p-toluene sulfonate is carried out in the presence of water and sodium hydroxide at a

temperature of from about 95 to about 100 °C.